# EUROPEAN PATENT SPECIFICATION

(11) **EP 0 423 282 B1**
(45) Date of publication and mention of the grant of the patent: **04.08.1993**
(21) Application number: 90906265.5
(22) Date of filing: 19.04.1990
(51) Int. Cl.: C07C 235/68, C07C 231/14

(54) **PROCESS FOR PREPARING N-PHENYL-N-METHOXYACETYL-DL-ALANINE-METHYLESTER DERIVATIVES**
VERFAHREN ZUR HERSTELLUNG VON N-PHENYL-N-METHOXYACETYL-DL-ALANIN-METHYLESTER-DERIVATEN
PROCEDE DE PREPARATION DE DERIVES DE N-PHENYL-N-METHOXYACETYL-DL-ALANINE-ESTER METHYLIQUE

(30) Priority: 19.04.1989 HU 190489
(43) Date of publication of application: 24.04.1991
(73) Proprietor: NITRO-KEMIA IPARTELEPEK, H-8184 Füzfögyartelep (HU)
(72) Inventor: AGOCS, Pal, H-8200 Veszprém (HU); PELYVA, Jenö, H-8184 Füzfögyártelep (HU); NAGY, Lajos, H-8184 Füzfögyártelep (HU); LEGRADI, Laszlo, H-8184 Füzfögyartelep (HU); KOLONICS, Zoltán, H-8220 Balatonalmádi (HU); SÖPTEI, Csaba, H-8200 Veszprém (HU); SEBÖK, Dezsö, H-8200 Veszprém (HU); MOLNAR, Sandor, H-8184 Füzfögyártelep (HU)
(74) Representative: von Füner, Alexander, Dr.
(86) International application number: HU9000027
(87) International publication number: WO9012783

(56) References cited:
- AT-B- 341 827
- CH-A- 639 643
- GB-A- 1 500 581
- GB-A- 2 024 211
- GB-A- 2 037 746
- GB-A- 2 070 584
- US-A- 3 048 626
- US-A- 3 780 090
- US-A- 4 317 916
- CHEMICAL ABSTRACTS, vol. 108, no. 1, 4 January 1988, Columbus, Ohio, USA; B- LIN et al, "Synthesis of metalaxyl from methyl 2-(2,6-xylidino)-propionate", see page 537, column 2, abstract no. 5695p, 6 CN-A-85 106 327

## Description

The invention relates to a process for preparing such N-phenyl-N-methoxyacetyl-DL-alanine-methylester derivatives, where the phenyl group may contain substituents in places 2, 4 and/or 6. Each substituent can be hydrogen atom or C₁₋₄ straight or branch chained alkyl group.

A known representative of N-phenyl-N-methoxyacetyl-DL-alanine-methylester derivatives is N-(2,6-di-methylphenyl)-N-methoxyacetyl-DL-alanine-methylester (commercial name: METALAXYL), a systemic fungicide, introduced by Ciba-Geigy AG in 1973.

The fungicidal characteristics thereof were described first by F. J. Schwin and al. (Mitt. Biol. Bundesanst. Land-Forstswirtsch. Berlin-Dahlem, 178, 145 /1977/). As fungicidal composition it is used against peronosporales delivered by air and soil. In case of subtropic and tropic culture plants it is used on the leaves in form of spray in a wide temperature range. It is recommended against Pseudoperonospora humili in hop, Phytophthora infestans in potato, Peronospora tabacina in tobacco and Plasmopara viticola damaging in grape plants. METALAXYL is also used against pathogenics living in the soil, causing rotting of the lower parts of the roots and stems by avocado and cytrus-type plants. It is used against rust in case of corn, pea, sorghum and sunflower, too.

METALAXYL contains asymmetric carbon atom so there exist two optical antipodes thereof. These are not separated from each other in the practice, so the active ingredient is the mixture of the two enantiomers (D and L). Though D-enantiomer has a stronger fungicidal activity as the L-form or DL-mixture, the industrial resolving is not economical.

On the basis of the known processes there are two different reaction methods for preparing METALAXYL. According to the CH-PS 607.888 and DE-PS 2,515.091 2,6-dimethyl-aniline is reacted with DL-α-bromo-propionic acid methylester in presence of sodium-hydrogencarbonate. The reaction results N-(2,6-dimethyl-phenyl)-DL-alanine-methylester. Acylation with methoxy-acetyl-chloride is then carried out and the end-product, METALAXYL is thus obtained according to reaction scheme A.

According to the other reaction route described in DE-PS 2,305.495 2,6-dimethyl-aniline is reacted with pyruvic acid-methylester. The obtained Schiff-base is hydrogenized catalytically and the obtained alkyl-derivative is acylated in a manner described above, when the end-product is formed according to reaction scheme B.

According to DE-PS 2,350.944 2,6-dimethylalanine is reacted with DL-α-bromo-propionic acid-methylester for 18 hours at a temperature of 120-125°C. The used alkylating agent excess is 3 fold, yield is of 79.6 % for this step.

In the processes described above acylation is carried out after the alkylation step. Acylation of the steric hindered secondary amine does not proceed so quickly as the acylation of the primary amine. Acid chloride should be used in great excess and the yield is moderate.

In US-PS 4,317.916 methoxyacetylchloride of a 10-fold molar excess is used. The excess acid chloride is then distilled off in vacuum and regenerated. It is not necessary to emphasize the elaborateness and high costs of this process.

The common feature of the processes mentioned is that the end product is prepared by the following steps in the following sequence:
1. alkylation
2. acylation

The invention relates to a process for preparing N-phenyl-N-methoxyacetyl-DL-alanine-methylester derivatives of the general formula (I) - wherein

R stands for a hydrogen atom or C₁₋₄ alkylgroup - in a way that an optionally substituted aniline of the general formula (II) - wherein R has the meaning as defined above - is acylated at first with the mixture of methoxyacetic acid used in excess of 5-20% and phosphorus trichloride in a solvent medium, then the obtained N-methoxyacetyl-aniline-derivative of the general formula (III) - wherein R has the same meaning as defined above - is alkylated immediately or after isolation with alkyl-haloide of an excess of 1.2 - 2.0 M in the presence of an alkali metal, alkali metal alkoholate, alkali metal amide or a complex metal hydride at a temperature of 100-140°C.

The process of the invention is based on the recognition that it is more advantageous to subject the aromatic primary amines at first to acylation and then to alkylation. In this case the procedures mentioned take place easier than according to the known processes - by alkylation at first and then by acylation.

Acylation takes place easier because primary amino group can be acylated easier than a steric hindered secondary amine. Alkylation is much quicker in this case for the mechanism of the procedure is completely different. Namely it was recognized that the acidic hydrogen atom on the nitrogen atom of methoxy-acetanilides substituted in positions 2, 4 and/or 6 can be replaced by alkali metals. The replacement can be carried out by alkali metals, alkali metal-alkoholates, alkali metal amides or complex metal hydrides. Salt-like compounds are thus obtained reacting easily with alkyl-halogenides, as with DL-α-bromo-propionic acid-methylester according to reaction scheme C.

Advantages of the process compared to the known methods are as follows;

The aniline substituted in the aromatic nucleus is acylated by using methoxy acetic acid + phosphorus trichroride.

In this case methoxy-acetyl-chloride is formed in situ then reacts with aniline. So one step, namely the preparation of methoxyacetylchloride is saved. For acylation of methoxyacetic acid with phosphorus-trichloride no reference has been found in the literature. Acylation is carried out in a solvent medium by using preferably aromatic solvents, toluene, chloro-benzene or xylene. Acylation takes place within 0.5 - 1.0 hour.

Alkylation is carried out with alkylhalogenide, preferably with halogen-carboxylic acid ester, so with DL-α-bromo-propionic acid-methylester. The reaction temperature can be varied between 100-140°C, preferably between 115-125°C. If alkylation is carried out in the present of alkali metal alkoholate the alkohol setting free can be removed preferably continuously from the reaction mixture.

By using the instant method - effecting alkylating after acylation - the addition of reactants requires 1 hour, the post reaction requires 30 minutes, the reaction proceeds completely and the product can be obtained with a yield of 90 %. The used DL-α-bromopropionic acid-methylester excess is only 2-fold.

Compared to the known methods the advantages of the process according to the invention can be summarized as follows:
1) A new synthesis method is described for preparing N-phenyl-N-methoxyacetyl-DL-alanine-methylester derivatives - e.g. the known fungicide "METALAXYL" according to which aromatic primary amine is at first acylated then alkylated. Thus both procedures take place more quickly and with a better yield than if alkylating is carried out at first and then acylating. Acylation takes place easier because primary amine can be acylated more easily than secondary amine, and the alkylation is also easier because the sodium salt of acylated amine is reacted with alkylhalogenide.
2) For acylation a methoxy-acetic acid + phosphorous trichlorid mixture is used instead of methoxyacetyl-chloride. Thus the preparation, isolation and regeneration of methoxyacetylchloride is saved.
3) By the instant acylation method the acylating agent is used in an excess of 10% compared to US-PS 4,317.916, where methoxyacetylchloride should be used in a 10-fold excess to achieve an appropriate yield.
4) According to DE-PS 2,350.944 DL-α-bromopropionic acid methylester is used in a 3-fold excess. In the process according to the invention the alkylating agent is used only in a 2-fold excess, the reaction time is 90 minutes compared to the 18 hours, and the yield obtained is by 10% higher.
5) According to the instant process acylation and alkylation can be carried out without isolating the intermediate - moreover this is more advantageous. This is also an important feature, because in the known processes the intermediate must be isolated and also purified.
6) The instant process protects the environment. As side product only poliphosphorous acid and sodium bromide are obtained which can preferably be used in other procedures as they are slightly contaminated. The xylene mother liquor obtained at the end of the procedure is of negligible quantity and can be burned. Sewege water will not be formed during the process.

### Example 1

### Preparation of N-methoxyacetyl-2,6-dimethyl aniline

Into a flask of 1 dm³ equipped with a stirrer, dropping funnel, thermometer and reflux condenser 500 cm³ of toluene, 121.19 g (1.0 M) of 2,6-dimethyl-aniline and 99.0 g (1.1 M) of methoxyacetic acid were measured. 50.6 g (0.37 M) of phosphorous trichloride dissolved in 100 cm³ of toluene was added dropwise within 1 hour under stirring. The system was then kept under reflux for 90 minutes while hydrochloric acid gas removed from the reaction mixture. The reaction mixture was cooled to room temperature and washed with water. Toluene was distilled of and the traces of the solvent were removed in vacuo. The residue was light yellow crystalline product.

Mp. 64°C. Yield: 183 g (94.6 %)

### Example 2

### Preparation of N-(2,6-dimethylphenyl)-N-(methoxyacetyl)-DL-alanine

Into a flask equipped with a stirrer, reflux, descending cooler, thermometer and feeding funnel 9.66g (0.05 M) of N-methoxyacetyl-2,6-dimethyl-aniline prepared according to Example 1, 60 cm³ of xylene and 16.7 g (0.1 M) of DL-α-bromopropionic acid methylester were added. The mixture was heated to 120°C and 20 cm³ of a 30 m% of sodium methylate was added drop-wise during 1 hour while distilling the methanol continuously from the reaction mixture. The temperature of the mixture must be not lower than 110°C. The reaction mixture was then stirred for further 30 minutes than cooled to room temperature. It was mixed with 100 cm³ of water, separated and the aqueous phase extracted with 3 x 20 cm³ of xylene. The xylene fractions were combined dried on sodium sulphate and the xylene was evaporated. The cooled residue solidifies into a crystalline substance.

Mp.: 73.5°C. Yield: 12.5-13.0 g (89.5-93.0%)

### Example 3

### Preparation of N-(2,6-dimethylphenyl)-N-(methoxy-acetyl)-DL-alanine-methylester from 2,6-dimethylaniline without isolating the intermediate.

6.06 g (0.05 M) of 2,6-dimethyl-aniline and 4.95 g (0.055 M) of methoxyacetic acid were dissolved in 30 cm³ of xylene and 2.6 g (0.019 M) of phophorous trichloride dissolved in 10 cm³ of xylene were added dropwise under stirring. After addition the mixture is kept under reflux for 30 minutes then cooled to room temperature. The xylene solution was separated from the polyphosphorous acid and 16.7 g (0.1 M) of DL-α-bromo-propionic acid methylester were added. Descending cooler equipped with a reflux condenser was fixed to the flask. The mixture was heated to 120°C and 20 cm³ of 30 vol% of sodium methylate were added dropwise during 1 hour while distilling off continuously the methanol from the reaction mixture. The temperature of the reaction mixture must not be lower than 110°C ! Thereafter the reaction mixture is stirred for further 30 minutes, then cooled to room temperature. The sodium bromide formed was filtered out from the solution and washed with 2 x 10 cm³ of xylene. The xylene parts were combined and the solution was evaporated to a volume of 20 cm³, then cooled to 0°C. The substance crystalled out was filtered.

M.p.: 73-74°C. Yield: 13.0 g (93.0 %)

Further product can be recovered from the obtained mother liquor.

### Example 4

### Preparation of N-methoxyacetyl-2,4,6-trimethyl-aniline

The process described in Example 1 was followed with the difference that 135.21 g of 2,4,6-trimethyl-aniline was used in the reaction.

M.p.: 88-89°C.

Yield: 188.85 g (92.0 %)

### Example 5

### Preparation of N-(2,4,6-trimethylphenyl)-N--methoxyacetyl-DL-alanine methylester

The process described in Example 2 was followed with the difference that 10.26 g of N-methoxyacetyl-2,4,6-trimethyl-aniline was used in the reaction. The end-product is a thick oil. Yield: 12.9 g (88.0 %)

### Example 6

### Preparation of N-(2-ethyl-phenyl)-N-methoxy-acetyl-DL-alanine-methylester

The process according to Example 3 was followed with the difference that 2-ethyl-aniline was used instead of 2,6-dimethyl-aniline.

The end-product was a thick oil.

Yield: 13.3 g (94.5 %).

### Example 7

### Preparation of N-(4-isopropyl-phenyl)-N-methoxyacetyl-DL-analine-methylester

The process described in Example 3 was followed with the difference that 4-isopropyl-aniline was used instead of 2,6-dimethyl-aniline.

The end-product was a thick oil.

Yield: 13.35 g (91.0 %).

### Example 8 (comparative)

### Preparation of N-(2,6-dimethyl-phenyl)-N-methoxyacetyl-DL-alanine-methylester according to CH-PS 607,888 (Ciba-Geigy).

Calculated to the starting 2,6-dimethyl-aniline the process can be carried out with a yield of 42 %.

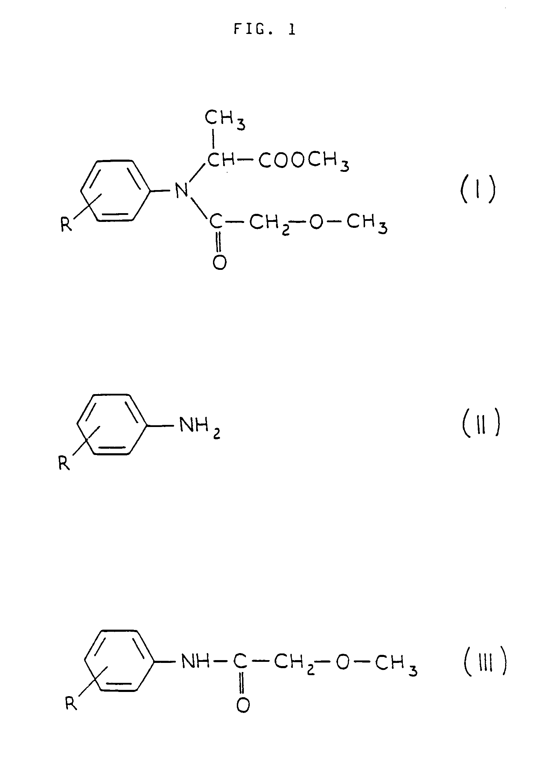

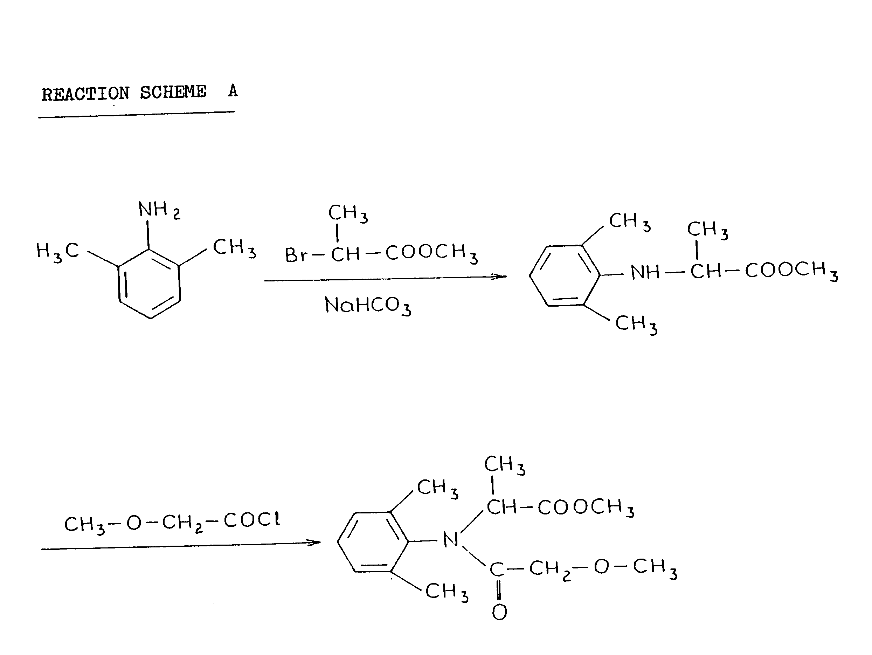

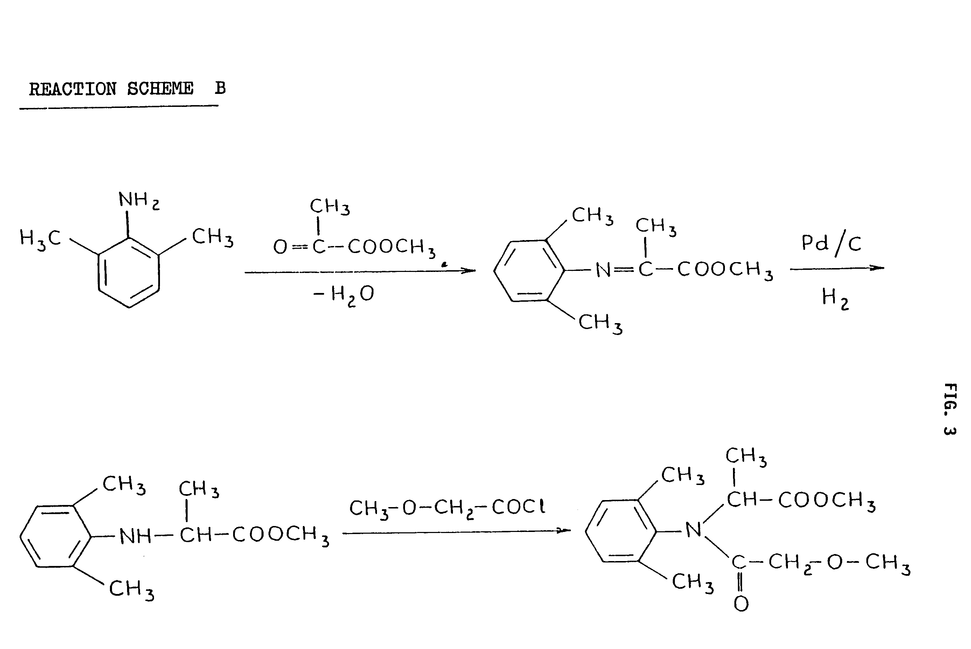

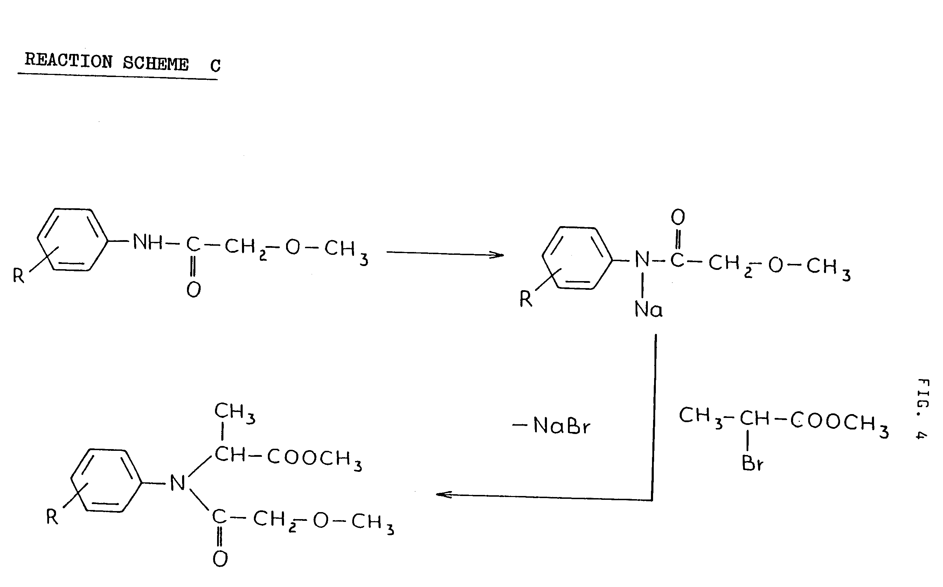

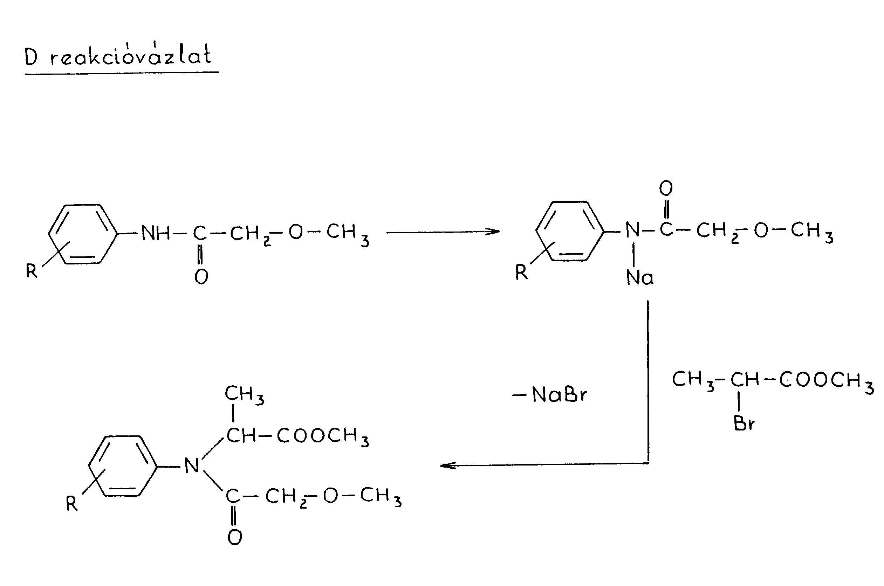

## Claims

1. Process for preparing N-phenyl-N-methoxy-acetyl-DL-alanine-methylester of the general formula (I) - wherein R stands for hydrogen atom or C₁₋₄ alkyl group, characterized in that the optionally substituted aniline of the general formula (II) - wherein R is as given above - is acylated at first with a mixture of methoxyacetic acid of an excess of 5-20 % and phosphorous trichloride in a solvent medium, then the obtained N-methoxyacetyl-aniline-derivative of the general formula (III) - wherein R has the same meaning as defined above - is alkylated immediately or after isolation with alkyl-halogenide of an excess of 1.2 - 2.0 M preferably with halogen carboxylic acid ester, in the presence of alkali metal, alkali metal-alkoholate, alkali metal amide or complex metal hydride at a temperature of 100-140°C.

2. A process according to claim 1 which. comprises carrying out the acylation and alkylation in quick succession.

3. A process according to claim 1 which comprises isolating the N-methoxyacetyl-aniline derivative of the general formula (III).

4. A process according to claim 1 which comprises carrying out the alkylation at a temperature between 115-125°C.

5. A process according to claim 1 characterized in that in case of using alkali metal alkoholate the alkohol is continuously removed from the reaction mixture.

6. A process according to claim 1 which comprises using as solvent aromatic hydrocarbon, preferably toluene or xylene.

## Patentansprüche

1. Verfahren zur Herstellung des N-Phenyl-N-methoxyacetyl-DL-alanin-methylesters der allgemeinen Formel I 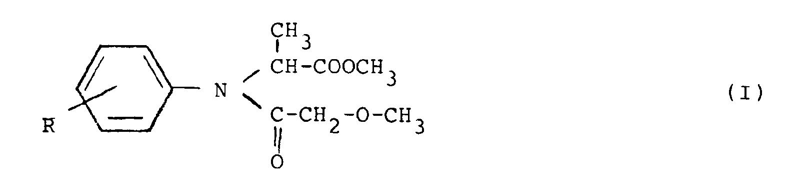 worin
R für ein Wasserstoffatom oder eine C₁₋₄-Alkylgruppe steht, dadurch **gekennzeichnet,** daß man eine gegebenenfalls substituiertes Anilin der allgemeinen Formel II 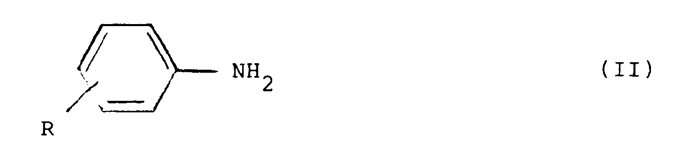 worin R die obige Bedeutung hat, zuerst mit einem Gemisch aus einem 5- bis 20%igen Überschuß an Methoxyessigsäure und Phosphortrichlorid in einem Lösungsmittel acyliert und dann das erhaltene N-methoxyacetyl-anilin-Derivat der allgemeinen Formel III 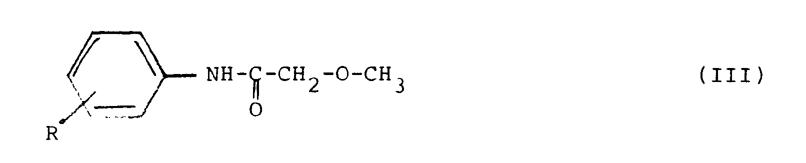 worin R die obige Bedeutung hat, unmittelbar danach oder nach Isolierung mit einem Überschuß von 1,2 bis 2,0 m eines Alkylhalogenids, vorzugsweise eines Halogencarbonsäureesters in Anwesenheit eines Alkalimetalls, Alkalimetallalkoholats, Alkalimetallamids oder eines komplexen Metallhydrids bei einer Temperatur von 100 bis 140°C alkyliert.

2. Verfahren nach Anspruch 1, bei dem die Alkylierung unmittelbar auf die Acylierung folgt.

3. Verfahren nach Anspruch 1, bei dem das N-methoxyacetylanilin-Derivat der allgemeinen Formel III isoliert wird.

4. Verfahren nach Anspruch 1, bei dem die Alkylierung bei einer Temperatur von 115 bis 125°C durchgeführt wird.

5. Verfahren nach Anspruch 1, dadurch **gekennzeichnet,** daß im Falle der Verwendung eines Alkalimetallalkoholats der Alkohol kontinuierlich aus dem Reaktionsgemisch entfernt wird.

6. Verfahren nach Anspruch 1, bei dem man als Lösungsmittel einen aromatischen Kohlenwasserstoff, vorzugsweise Toluol oder Xylol, verwendet.

## Revendications

1. Procédé pour la préparation de méthylester de N-phényl-N-méthoxyacétyl-DL-alanine de formule générale (I) dans laquelle:
R représente un atome d'hydrogène ou un groupe alkyle en C₁ à C₄
caractérisé en ce que l'alanine, éventuellement substituée, de formule générale (II), dans laquelle R a la signification donnée ci-dessus, est acylée en premier par un mélange d'acide méthoxyacétique en un excès de 5 à 20% et de trichlorure de phosphore dans un solvant, le dérivé de N-phényl-N-méthoxyacétylalanine ainsi obtenu, de formule générale (III), dans laquelle R a la même signification que celle donnée ci-dessus, étant alkylé immédiatement, ou après séparation par un halogénure d'alkyle employé en un excès de 1,2 à 2,0 molaire, de préférence par un ester d'acide halogénocarboxylique, en présence d'un métal alcalin, d'un alcoolate de métal alcalin, d'un amide de métal alcalin ou d'un hydrure de métal complexe, à une température de 100 à 140°C.

2. Un procédé selon la revendication 1, caractérisé en ce que l'acylation et l'alkylation sont effectuées selon une succession rapide.

3. Un procédé selon la revendication 1, caractérisé en ce qu'il comprend la séparation du dérivé de N-méthoxyacétylaniline de formule générale (III).

4. Un procédé selon la revendication 1, caractérisé en ce que l'alkylation est effectuée à une température comprise entre 115 et 125°C.

5. Un procédé selon la revendication 1, caractérisé en ce que, dans le cas où on utilise un alcoolate de métal alcalin, l'alcool est éliminé en continu du milieu réactionnel.

6. Un procédé selon la revendication 1, caractérisé en ce que l'on emploie comme solvant un hydrocarbone aromatique, de préférence du toluène ou du xylène.
